# EUROPEAN PATENT APPLICATION

(11) **EP 0 696 736 A1**
(43) Date of publication of application: **14.02.1996**
(21) Application number: 95112435.3
(22) Date of filing: 08.08.1995
(51) Int. Cl.: G01N 33/564, G01N 33/543, G01N 33/541

(54) **Detection of high risk factor to develop cataract and a reagent kit for the detection**

(30) Priority: 10.08.1994 JP 188451/94
(71) Applicant: Senju Pharmaceutical Co., Ltd., Osaka-shi, Osaka 541 (JP)
(72) Inventor: Azuma, Mitsuyoshi, Nishinomiya-shi, Hyogo 662 (JP); Inoue, Eri, Himeji-shi, Hyogo 671-02 (JP); Oka, Takayuki, Kobe-shi, Hyogo 651-21 (JP)
(74) Representative: von Kreisler, Alek, Dipl.-Chem.

(57) **Abstract**

A method for detecting a high risk factor which develops cataract, comprising assaying an autoantibody against a lens specific antigen in a human body fluid by an immunoassay using a lens specific antigen, and a reagent kit for detecting a high risk factor which develops cataract, the kit comprising a lens specific antigen. The detection method and the reagent kit for the detection of the present invention enable safe, easy and economical detection of a high risk factor which develops cataract in individuals who appear normal at present but may possibly suffer from cataract in the future, let alone patients in the initial stage of cataract. Accordingly, they can be advantageously used not only for the detection of cataract but for early detection of individuals having a high risk of developing cataract in the future, early treatment of cataract and prevention thereof.

## Description

### FIELD OF THE INVENTION

The present invention relates to an immunological method for detecting a high risk factor to develop cataract in the future and a reagent kit for the detection.

### BACKGROUND OF THE INVENTION

Cataract is a disease wherein the lens becomes opaque due to various causes, which comes out in the form of paropsis caused by the opacity of the lens.

According to the causes, cataract is classified into stationary cataract, senile cataract, complicated cataract, traumatic cataract, diabetic cataract, radiation cataract etc., all of which constitute serious eye diseases which accompany visual loss. Yet, the cause of senile cataract, which is the majority of these cataracts, is still unknown.

Cataract has so far been treated by the administration of various pharmaceutical agents. However, administration of such agents is not entirely satisfactory partly due to the markedly slow metabolism of the lens. Consequently, the treatment of cataract is currently limited to surgical ones. The surgical treatment is given by removal of the lens nucleus and lens cortex to outside the membrane of the lens, or enucleation of the entire opaque lens out from the eye. However, surgical operations often induce complications and they are burdensome to patients in that incision and suture of the cornea are inevitable even when operated with the use of the state-of-the-art surgery technique.

In recent years, it has been reported that the cause of cataract may be an autoimmune phenomenon [(1) Angunawela, I.I., Immunology, *61*, 363-368 (1987); (2) Niederkorn, J.Y., Immunology Today, vol. 8, No. 11, 332-333 (1987)].

Of these, Angunawela reported a significant increase in the production of autoantibodies against lens antigens in the sera of patients having cataractous lens and in the sera of patients with diabetes, as well as immunoglobulin detectable in cataractous lenses. However, antibody titer was not determined in the Angunawela report, and the possibility of increased antibody titer of autoantibody becoming an indicator to teach the risk of developing cataract in the future has not been indicated. In addition, the Angunawela report does not specifically identify the kind of the lens protein antigen.

Under the circumstances, the present inventors considered the advantageous aspect of a method for detecting cataract and a detection kit therefor which are capable of pointing out individuals who are seemingly normal at present but may possibly develop cataract in the future, for the early detection of individuals having a high risk of developing cataract in the future, not to mention early detection and early treatment of cataractous patients, and done an intensive study.

As a result, the present inventors have found that an autoantibody against a lens specific antigen in a human body fluid is a high risk factor which develops cataract in individuals who are seemingly normal at present but may possibly contract cataract in the future, and that detection of the high risk factor enables finding individuals having a high risk of developing cataract in the future.

### SUMMARY OF THE INVENTION

It is therefore an object of the present invention to provide a method for detecting a high risk factor which develops cataract, comprising assaying an autoantibody against a lens specific antigen in a human body fluid by an immunoassay using a lens specific antigen.

In particular, the present invention provides the above-mentioned detection method comprising the following steps of immunoassay:
(a) reacting an autoantibody against a lens specific antigen in a human body fluid with a lens specific antigen and
(b) assaying the autoantibody against the lens specific antigen using a secondary antibody against human immunoglobulin.

The present invention also relates to a reagent kit for detecting a high risk factor which develops cataract, the kit comprising a lens specific antigen.

The present invention further relates to a method for detecting cataractous patients or individuals who may possibly develop cataract, comprising assaying an autoantibody against a lens specific antigen in the body fluid of subjects by an immunoassay using a lens specific antigen.

### BRIEF DESCRIPTION OF THE DRAWING

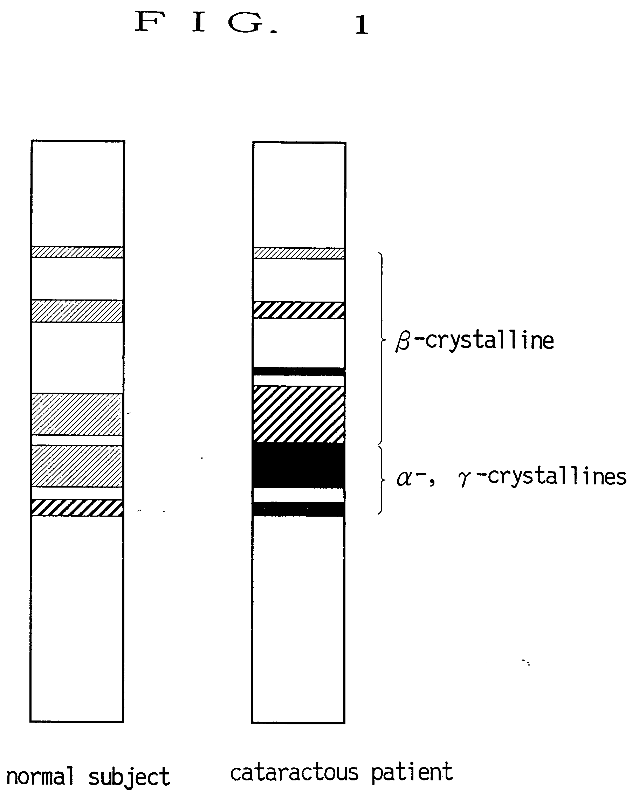

Fig. 1 shows typical immunoblot patterns of a normal subject and a cataractous patient.

### DETAILED DESCRIPTION OF THE INVENTION

In the present invention, immunoassay is not particularly limited as long as it can assay an antibody specific to an antigen, utilizing an antigen-antibody reaction, and can be a known method. Examples thereof include known methods such as immunoblot (western blot) assay, enzyme immunoassay (ELISA), agglutination reactions (e.g. latex agglutination and passive hemagglutination), radioimmunoassay and fluorescent/light emitting immunoassay. In view of high sensitivity and easy and simple operation, preferred are immunoblot assay, enzyme immunoassay and agglutination reactions.

The lens specific antigen to be used in the present invention can be a lens crystalline (e.g. α-crystalline, β-crystalline and γ-crystalline), a membrane protein of lens, a receptor thereof, a lens homogenate or an epitheliocyte homogenate.

The human body fluid to be used as a sample in the present invention may be any insofar as it allows detection of autoantibody therein, and is exemplified by blood, plasma, serum, ascites, lymph, intra-articular fluid, saliva, tear, fractions obtained therefrom and liquid components derived from other biological sources, with preference given to serum from human peripheral blood.

The detection method of the present invention is described in detail in the following by way of examples.

### (1) Immunoblot

The immunoblot assay comprises reacting an autoantibody against a lens specific antigen in a human body fluid with a lens specific antigen blotted onto a membrane filter, adding a conjugated secondary antibody against human immunoglobulin, and assaying the conjugated secondary antibody bound to the autoantibody.

### ① Blotting of antigen

A lens specific antigen can be blotted by a conventional method. For example, the lens specific antigen is separated by the method of Laemmli [Laemmli et al., Nature, vol. 227, pp 680-687, UK (1970)] by sodium dodecyl sulfate polyacrylamide gel electrophoresis (SDS-PAGE). Then, the obtained lens specific antigen is blotted onto a membrane filter according to the method of Towbin et al. [Towbin H., Staehelin, T. and Gordon, J., Proc. Natl. Acad. Sci. vol. 76, No. 9, pp 4350-4354, USA (1979)]. As the membrane filter, usable are nitrocellulose filter, Immobilon polyvinylidene difluoride (PVDF) and the like. The membrane filter onto which the lens specific antigen has been blotted is cut into, for example, long strips of about 0.5 cm width and 10 cm length and subjected to the reaction with human body fluid.

The membrane filter onto which the lens specific antigen has been blotted is preferably blocked to prevent nonspecific adsorption, using a suitable blocking solution such as a buffer [e.g. phosphate buffered saline (PBS) and Tris buffered solution (TBS)] containing 1-3% bovine serum albumin (BSA).

### ② Reaction with primary antibody

The following reactions and washing are preferably performed on a commercially available mini-incubation tray such as those available from Bio-Rad. A tray with a lid on is preferable, since it is free from contamination or water evaporation during storage and incubation. The tray and lid are preferably made from an acrylic plastic.

The human body fluid to be used as a test sample is diluted about 10- to 100-fold before incubation. The membrane filter onto which the antigen has been blotted is incubated with about 0.5 - 1 ml of a diluted sample on the tray while minimizing exposure to biohazardous materials, whereby the autoantibody (primary antibody) against the lens specific antigen in the test sample is reacted with the lens specific antigen. Incubation is performed at 4-37°C for 1-24 hours, preferably about 16 hours. After reaction, the reaction mixture is removed and the membrane filter is washed.

### ③ Reaction with secondary antibody

The secondary antibody against human immunoglobulin is an antibody capable of binding to an autoantibody to be assayed, and can be an antibody conjugated with enzyme, radioactive isotope, fluorescent substance, light emitting substance or other labeling substance, with preference given to a secondary antibody conjugated with an enzyme. Examples of the secondary antibody include goat anti-human immunoglobulin, rabbit anti-human immunoglobulin and sheep anti-human immunoglobulin, all conjugated with a labeling substance such as peroxidase, alkaline phosphatase and β-galactosidase. The secondary antibody is added and the mixture is reacted at 20-37°C for about 1 to 2 hours. The secondary antibody is preferably diluted 1,000- to 5,000-fold with a blocking solution and put to use. After the completion of the reaction, the reaction mixture is removed and the membrane filter is washed.

### ④ Assay of secondary antibody

The secondary antibody can be assayed by a known method according to the kind of the labeling substance used for the secondary antibody. When an enzyme-conjugated secondary antibody is used, a known color developing agent corresponding to the selected enzyme is added to cause color development. For example, when peroxidase is used, a substrate such as o-phenylenediamine and ABTS [2,2'-azino-bis(3'-ethylbenzodiazolinesulfonic acid)] and hydrogen peroxide is used as a color developing agent; when alkaline phosphatase is used, a substrate such as p-nitrophenylphosphoric acid is used as a color developing agent; and when β-galactosidase is used, a substrate such as o-nitrophenyl-β-D-galactoside and 4-methylumbellipheryl-β-D-galactoside is used as a color developing agent. As a result, a color development dependent on the amount of autoantibody in a sample is achieved. Color depth is compared with that of the standard sample having a known antibody titer, whereby the autoantibody in the test sample is quantified.

### (2) Enzyme immunoassay

Immunoassay by an enzyme immunoassay comprises reacting an autoantibody against a lens specific antigen in a human body fluid with a lens specific antigen immobilized on a solid phase, adding a conjugated secondary antibody against human immunoglobulin, and assaying the conjugated secondary antibody bound to the autoantibody.

### ① Immobilization of antigen

A lens specific antigen is immobilized on a solid phase. Examples of the solid phase include polyvinyl chloride, polystyrene, polyethylene, polypropylene, polyester, fluororesin, glass and metal. The shape of the carrier is, for example, plate, beads, cell and test tube. Preferred is a commercially available, known solid phase, such as a microtiter plate (e.g. 96 well microtiter plate) for enzyme immunoassay.

The method for immobilizing the antigen may be known. For example, an antigen solution dissolved in a suitable buffer is brought into contact with a solid phase, and left standing at 4-37°C for 1-24 hours to immobilize the antigen. When using a polyvinyl chloride (PVC) microtiter plate, about 100 ng/well antigen is bound to the plate. It is preferable that the unadsorbed portion be blocked using a blocking solution such as a buffer containing 1-3% BSA [e.g. phosphate buffered saline (PBS) and Tris buffered solution (TBS)] to prevent nonspecific adsorption. The solid phase is washed with an appropriate buffer solution. By the above-mentioned steps, the antigen is immobilized.

### ② Reaction with primary antibody

The human body liquid to be used as a test sample is diluted about 100-fold to 5,000-fold before incubation. The diluted sample is added to the solid phase prepared above and incubated, whereby the autoantibody (primary antibody) against the lens specific antigen in the test sample is reacted with the lens specific antigen. Incubation is performed at 4-37°C for 1-24 hours, preferably 16 hours. After the completion of the reaction, the reaction mixture is removed and the solid phase is washed.

### ③ Reaction with secondary antibody

The secondary antibody against human immunoglobulin is capable of binding to the autoantibody to be assayed, and those conjugated with enzyme, radioactive isotope, fluorescent substance, light emitting substance or other labeling substance can be used, with preference given to a secondary antibody conjugated with an enzyme. Examples of the secondary antibody include goat anti-human immunoglobulin, rabbit anti-human immunoglobulin and sheep anti-human immunoglobulin, all conjugated with a labeling substance such as peroxidase, alkaline phosphatase and β-galactosidase. The secondary antibody is added to the solid phase and the mixture is reacted at 4-37°C for about 1 to 2 hours. The secondary antibody is preferably diluted 1,000- to 5,000-fold and subjected to the reaction. After the completion of the reaction, the reaction mixture is removed and the solid phase is washed.

### ④ Assay of secondary antibody

The secondary antibody can be assayed by a known method according to the kind of the labeling substance used for the secondary antibody. When an enzyme-conjugated secondary antibody is used, a known color developing agent corresponding to the enzyme selected is added to cause color development. For example, when peroxidase is used, a substrate such as o-phenylenediamine and ABTS [2,2'-azino-bis(3'-ethylbenzodiazolinesulfonic acid)] and hydrogen peroxide is used as a color developing agent; when alkaline phosphatase is used, a substrate such as p-nitrophenylphosphoric acid is used as a color developing agent; and when β-galactosidase is used, a substrate such as o-nitrophenyl-β-D-galactoside and 4-methylumbellipheryl-β-D-galactoside is used as a color developing agent. As a result, a color development dependent on the amount of autoantibody in the sample is achieved. The absorbance of the reaction mixture is determined using a spectrophotometer or fluorophotometer, based on which the autoantibody in the test sample is quantified.

### (3) Agglutination reaction

In an immunoassay using agglutination reaction, carrier particles bound with a lens specific antigen are used. Examples of the particles include polystyrene latex, kaolin, bentonite, carbon powder and erythrocytes of sheep, chicken and the like. A known method is used for binding the antigen to the carrier particles. For example, a coupling agent such as tannic acid, glutaraldehyde, bisazobenzidine, carbodiimides, quinones and chromium chloride is used, or physical adsorption is performed.

The agglutination reaction can be performed according to the method conventionally used for latex agglutination and passive agglutination. For example, a serially diluted test sample (e.g. serum from human peripheral blood) is placed in the wells of a microplate, mixed with the above-mentioned particle carrier bound with lens specific antigen, and left standing for 1 or 2 hours to allow agglutination. The agglutination is compared with that of the standard test sample having a known antibody titer to quantify the autoantibody in the sample.

The reagent kit of the present invention comprises a lens specific antigen. The reagent kit of the present invention preferably comprises (a) a lens specific antigen, (b) a secondary antibody against human immunoglobulin and (c) a color developing agent for assaying the secondary antibody. The reagent kit comprising (a) to (c) is used for enzyme immunoassay (ELISA) or immunoblot assay. In the case of the reagent kit for enzyme immunoassay, the lens specific antigen may be immobilized on a solid phase in advance, or may be immobilized on a solid phase when in use. In this case, the reagent kit may comprise a solid phase to immobilize the antigen. In the case of a reagent kit for immunoblot assay, the lens specific antigen may be blotted onto a membrane filter in advance, or may be blotted onto a membrane filter when in use. In this case, the reagent kit may comprise a membrane filter for blotting the antigen. Preferably, a membrane filter onto which an antigen has been blotted is used. In the case of a reagent kit for agglutination reaction, it comprises carrier particles bound with a lens specific antigen. Besides the above-mentioned construction, the reagent kit may comprise a standard test sample having a known antibody titer, buffer, diluent, washing solution, reaction terminator and the like. Each component mentioned above can be prepared into a suspension, solution or lyophilized product.

The detection method and the reagent kit for the detection of the present invention enable safe, easy and economical detection of a high risk factor which develops cataract in those who appear normal at present but may possibly suffer from cataract in the future, let alone patients in the initial stage of cataract, by assaying an autoantibody against a lens specific antigen in a body fluid. In particular, an autoantibody against lens specific antigen, such as crystalline and membrane component, can be qualitatively assayed according to the detection method using immunoblot assay. Moreover, an autoantibody against a lens specific antigen can be quantitatively assayed according to the detection method using enzyme immunoassay or agglutination reaction. Hence, a combination of the immunoblot assay and enzyme immunoassay or agglutination reaction results in qualitative and quantitative assay of autoantibody against lens specific antigen, and a treatment method for preventing the progress of cataract can be determined.

The present invention is described in more detail by way of Examples, and the effects of the present invention are clarified by Experimental Examples. It should be understood that these are mere exemplifications and the scope of the present invention is not limited thereby.

### Example 1

A human lens homogenate was prepared by homogenizing human lens and buffer in a homogenizer and used as a human lens specific antigen.

The human lens specific antigen (10 µg/ml) was diluted with Dulbeco's phosphate buffered saline without calcium and magnesium, and placed in each well of a 96 well microtiter plate by 25 µl. Then, a solution (25 µl, pH 9.3) of sodium hydrogencarbonate containing 5 mM disodium ethylenediaminetetraacetate was added. The plate was shaken and left standing overnight at 4°C to allow the antigen to adsorb to the bottom surface of the wells. The antigen solution was removed and a blocking solution [100 µl, phosphate buffered saline (PBS, pH 7.0) containing 1% BSA] was added to each well. The plate was left standing at 4°C for 1 hour to cover the bottom of the wells (antigen-unadsorbed portion), and the solution was removed.

Diluted human serum (100 µl) was added to each well and the mixture was reacted overnight at 4°C. The diluted human serum was prepared by diluting human serum 250-fold, 750-fold or 2250-fold with a blocking solution. After the reaction, the diluted serum was removed and the wells were washed three times with PBS. A secondary antibody conjugated with alkaline phosphatase (alkaline phosphatase-conjugated goat anti-human immunoglobulin, manufactured by Bio-Rad) was diluted 2500-fold with a blocking solution and added by 100 µl. The mixture was reacted at 37°C for 2 hours. After the completion of the reaction, the plate was washed 4 or 5 times with PBS.

After final washing with PBS, a substrate solution (100 µl) was added to each well and the mixture was reacted for 30 minutes. The substrate solution was prepared by diluting diethanolamine buffer 5-fold with distilled water and dissolving p-nitrophenylphosphoric acid (5 mg tablet) (alkaline phosphatase substrate kit, manufactured by Bio-Rad). After the completion of the reaction, absorbance at 405 nm was determined using a plate reader (manufactured by Labsystems).

### Example 2

In the same manner as in Example 1, a human lens homogenate was prepared and used as a lens specific antigen. According to the method of Laemmli [Laemmli et al., Nature, vol. 227, pp 680-687, UK (1970)], the lens specific antigen was separated on one dimensional sodium dodecyl sulfate polyacrylamide gel electrophoresis (SDS-PAGE). SDS-PAGE was performed on discontinuous, 8% gels. Then, the obtained lens specific antigen was blotted onto a nitrocellulose filter according to the method of Towbin et al. [Towbin H., Staehelin, T. and Gordon, J., Proc. Natl. Acad. Sci. vol. 76, No. 9, pp 4350-4354, USA (1979)]. This membrane filter was cut into long strips of 0.5 cm width and 10 cm length.

The antigen-blotted membrane filter was placed in each row of a disposable 8 row tray (manufactured by Bio-Rad). Tris buffered solution (TBS, 2 ml) containing 2% BSA was added, and the plate was left standing at room temperature for 2 hours to cover the membrane filter surface (antigen-unadsorbed portion), and the solution was removed. The plate was washed for 5 minutes with TTBS (TBS containing 0.05% Tween 20). The washing was repeated twice. The human serum was used upon 100-fold dilution with TTBS containing 1% BSA prior to reaction.

Diluted human serum (1 ml) was added to each row of the tray, and the membrane filter was reacted overnight at 4°C. After the reaction, the diluted serum was removed and the membrane filter was washed for 5 minutes with TTBS. Then, the washing was repeated twice. A secondary antibody conjugated with alkaline phosphatase (alkaline phosphatase-conjugated goat anti-human immunoglobulin, manufactured by Bio-Rad) was diluted 2500-fold with TTBS containing 1% BSA and added by 1 ml. The membrane filter was reacted at room temperature for 2 hours. After the completion of the reaction, the membrane filter was washed twice with TTBS for 5 minutes each time, and twice with TBS for 5 minutes each time.

An alkaline phosphatase color developing kit (manufactured by Bio-Rad) was used for color development. The color developing solution (1 ml) was added to each row of the tray to allow color development of the membrane filter (blue-purple) and distilled water was added to stop the reaction. The color developing solution was prepared by mixing BCIP (5-bromo-4-chloro-3-indolyl phosphate) and NBT (nitroblue tetrazolium) with color development buffer.

### Experimental Example 1 : Assay of antibody specific to lens protein in human sera

### Test Method

Blood (5 ml) was taken from normal subjects (8 males, 43-65 of age) and 10 cataractous patients (8 females and 2 males, 41-78 of age). By normal subject is meant those showing no symptoms of cataract. The sera therefrom were diluted 250-fold with PBS containing 1% BSA and treated in the same manner as in Example 1 above and subjected to determination of absorbance at 405 nm to assay the amount of antibody against the lens protein. The results are shown in Table 1.

**Table 1**

| No. | Age | Sex | Normal subject Absorbance (A405) |
|---|---|---|---|
| 1 | 65 | male | 0.356 |
| 2 | 44 | male | 0.273 |
| 3 | 64 | male | 0.205 |
| 4 | 46 | male | 0.250 |
| 5 | 56 | male | 0.204 |
| 6 | 59 | male | 0.396 |
| 7 | 49 | male | 0.354 |
| 8 | 43 | male | 0.440 |
| | | | |

| No. | Age | Sex | Cataractous patient Absorbance (A405) |
|---|---|---|---|
| 1 | 71 | male | 0.982 |
| 2 | 65 | female | 0.723 |
| 3 | 73 | female | 0.556 |
| 4 | 63 | female | 0.666 |
| 5 | 41 | female | 0.504 |
| 6 | 78 | female | 0.996 |
| 7 | 63 | female | 0.980 |
| 8 | 56 | female | 0.739 |
| 9 | 58 | male | 0.946 |
| 10 | 73 | female | 0.720 |
| Note: In the Table, absorbance was measured at 405 nm using the serum diluted 250-fold. | | | |

### Test Results

As shown in Table 1, both the normal subjects and the cataractous patients had antibodies against the lens proteins in sera. However, the sera of all 10 cataractous patients showed higher values than normal subjects.

The results of Table 1 are indicative of the presence of antibody against the lens protein in normal subjects. The antibody increases as cataract develops. Accordingly, the antibody is a high risk factor which develops cataract in those who look normal at present but may possibly suffer from cataract in the future.

Based on the test results of Table 1, subjects showing absorbance of 250-fold diluted serum of over 0.5 were evaluated as positive and those showing an absorbance of not more than 0.5 were evaluated as negative. The evaluation results are shown in Table 2, wherein each figure denotes the number of subjects.

**Table 2**

| | Positive | Negative | (Total) |
|---|---|---|---|
| Normal subject | 0 | 8 | 8 |
| Cataractous patient | 10 | 0 | 10 |
| (Total) | 10 | 8 | 18 |

### Experimental Example 2 : Analysis of antibodies specific to α-, β- and γ-crystallines in human sera

### Test Method

In the same manner as in Experimental Example 1, blood was taken from normal subjects (8 males, 43-65 of age) and 10 cataractous patients (8 females and 2 males, 41-78 of age). According to the method of Example 2 above, it was examined to find that the antibodies against the lens protein in sera, which showed increase in Experimental Example 1, were antibodies against crystalline.

### Test Results

A typical immunoblot of a normal subject and a cataractous patient is shown in Fig. 1. As is evident from the results shown in Fig. 1, antibodies against α-, γ- and β-crystallines were present in the sera of both normal subjects and cataractous patients. The deeper color developed by the crystalline bands in the cataractous patient than that of the normal subject suggests higher antibody titer against α-, γ- and β-crystallines in the sera of cataractous patients, as in Experimental Example 1. Such results are indicative of the presence of antibodies against α-, γ- and β-crystallines in both the cataractous patients and the normal subjects. Such antibodies are high risk factors which develop cataract in individuals who look normal at present but may possibly suffer from cataract in the future.

The detection method and the reagent kit for the detection of the present invention enable safe, easy and economical detection of a high risk factor which develops cataract in individuals who appear normal at present but may possibly suffer from cataract in the future, let alone patients in the initial stage of cataract. Accordingly, they can be advantageously used not only for the detection of cataract but for early detection of individuals having a high risk of developing cataract in the future, early treatment of cataract and prevention thereof. In addition, such detection can be added to the current hematological check items.

## Claims

1. A method for detecting a high risk factor which develops cataract, comprising assaying an autoantibody against a lens specific antigen in a human body fluid by an immunoassay using a lens specific antigen.

2. The method of Claim 1, wherein the immunoassay comprises the following steps:
(a) reacting an autoantibody against a lens specific antigen in a human body fluid with a lens specific antigen and
(b) assaying the autoantibody against the lens specific antigen using a secondary antibody against human immunoglobulin.

3. The method of Claim 1, wherein the immunoassay comprises the following steps:
(a) reacting an autoantibody against a lens specific antigen in a human body fluid with a lens specific antigen immobilized on a solid phase,
(b) adding a conjugated secondary antibody against human immunoglobulin, and
(c) assaying the conjugated secondary antibody bound to the autoantibody.

4. The method of Claim 1, wherein the immunoassay comprises the following steps:
(a) reacting an autoantibody against a lens specific antigen in a human body fluid with a lens specific antigen blotted onto a membrane filter,
(b) adding a conjugated secondary antibody against human immunoglobulin, and
(c) assaying the conjugated secondary antibody bound to the autoantibody.

5. The method of Claim 1, wherein the immunoassay comprises the following steps:
(a) reacting an autoantibody against a lens specific antigen in a human body fluid with a lens specific antigen immobilized on carrier particles and
(b) assaying an agglutination caused by the reaction between the lens specific antigen and the autoantibody.

6. A reagent kit for detecting a high risk factor which develops cataract, the kit comprising a lens specific antigen.

7. The kit of Claim 6, comprising
(a) a lens specific antigen,
(b) a conjugated secondary antibody against human immunoglobulin, and
(c) a color developing agent for assaying the conjugated secondary antibody.

8. The kit of Claim 7, wherein the lens specific antigen is immobilized on a solid phase.

9. The kit of Claim 7, wherein the lens specific antigen is blotted onto a membrane filter.

10. The kit of Claim 6, wherein the lens specific antigen is bound to carrier particles.

11. A method for detecting cataractous patients or individuals who may possibly develop cataract, comprising assaying an autoantibody against a lens specific antigen in a body fluid of a subject by an immunoassay using a lens specific antigen.
